# EUROPÄISCHE PATENTANMELDUNG

(11) **EP 1 111 371 A2**
(43) Veröffentlichungstag der Anmeldung: **27.06.2001**
(21) Anmeldenummer: 00127902.5
(22) Anmeldetag: 20.12.2000
(51) Int. Cl.: G01N 21/37, G01N 21/35, A61B 5/083

(54) **Verfahren und Einrichtung zur Kalibrierung einer Messeinrichtung**

(30) Priorität: 23.12.1999 DE 19962588
(71) Anmelder: ABB PATENT GmbH, 68309 Mannheim (DE)
(72) Erfinder: Joksch, Burkhard, Dr. rer.nat., 61250 Usingen (DE); Liedtke, Thomas, Dipl.-Phys., 61350 Bad Homburg (DE); Fabinski, Walter, Dipl.-Ing., 65830 Kriftel (DE)
(74) Vertreter: Schmidt, Karl Michael, Dipl.-Phys.

(57) **Zusammenfassung**

Die Erfindung betrifft ein Verfahren sowie eine Einrichtung zur Kalibrierung einer Meßeinrichtung, insbesondere eines nichtdispersiven Infrarotspektrometers zur Atemgasanalyse, mit einem Strahlengang, bspw. mit einem 12 C02 und 13 C02 Kanal, in welchem die besagten Gaskomponenten selektiv gemessen werden können. Um ein Verfahren sowie eine Einrichtung der gattungsgemäßen Art dahingehend zu verbessern, daß trotz Trägergasabhängigkeit mit einer noch höheren Genauigkeit und Reproduzierbarkeit gemessen werden kann, ist erfindungsgemäß vorgeschlagen, daß in einem 1. Verfahrensschritt Atemgasteilproben mit unterschiedlicher Konzentration der zu messenden Gaskomponente und entsprechend der Trägergaskomponente durch unterschiedliche Atemtiefe erzeugt werden, und daß in einem 2. Verfahrensschritt eine Korrekturkurve aus den unterschiedlichen Werten der verschiedenen Atemgasteilproben ermittelt wird, und im nachfolgenden Messlauf die so erhaltene Korrekturkurve zur Eliminierung der Trägergasabhängigkeit herangezogen wird.

## Beschreibung

Die Erfindung betrifft ein Verfahren sowie eine Einrichtung zur Kalibrierung einer Meßeinrichtung, insbesondere eines nichtdispersiven Infrarotspektrometers zur Atemgasanalyse, mit einem Strahlengang, bspw. mit einem 12 CO2 und 13 CO2 Kanal, in welchem die besagten Gaskomponenten selektiv gemessen werden können.

Die nichtdispersive Infrarotspektroskopie ist in der Gasanalyse ein mittlerweile weitverbreitetes Verfahren. Die Messaufgabe besteht in nahezu allen Fällen, mit hoher Selektivität eine Messgaskomponente in einem Gasgemisch quantitativ erfassen zu können. Durch die hohe Empfindlichkeit und die hohe Selektivität, die mit diesem Verfahren mittlerweile beherrscht werden, werden Nebeneffekte wie Querempfindlichkeiten zwischen verschiedenen Gaskomponenten in einem Gasgemisch plötzlich wesentlich, weil die Empfindlichkeit bis hin zu extrem kleinen Konzentrationen reicht. Ein Beispiel hierfür ist in der DE 195 38 431 angegeben.

Eine wesentliche Querempfindlichkeit besteht in vielen Fällen zu Wasserdampf, der in den meisten Messgasen unvermeidlich ist. Hierzu werden vielfach die Störeinflüsse des Wasserdampfes in irgendeiner Weise quantifiziert, und das Messsignal damit korrigiert.

Eine weitere Problematik stellt die Trägergasabhängigkeit insbesondere zu Sauerstoff dar. Bei der Anwendung der nichtdispersiven Infrarotspektroskopie in der Medizin, besteht ein solche Trägergasabhängigkeit insbesondere zwischen der Messkomponente CO₂ und Sauerstoff.

Ein besagter Anwendungsfall in der Medizin betrifft die Atemgasanalytik von stabilen Isotopen wie 13 CO2 und in 12 CO2. Hierbei entstehen Abweichungen des Messergebnisses in Abhängigkeit der begleitenden Gaskomponenten, insbesondere des Sauerstoffgehaltes. Der Effekt beruht vornehmlich auf der Stoßverbreiterung von Rotationslinien. Hinzu kommt ein Volumenfehler durch den anteiligen Wasserdampfgehalt, der sich beim Ausatmen bei 37°C und der Messung bei Raumtemperatur ergibt. Da der Wasserdampfanteil durch sich ergebende Kondensationseffekte vom Probanden bis zum Analysator nicht konstant verhält, ensteht der oben genannte Volumenfehler. Die dadurch entstehende Abweichung beträgt bei Quotientenbildung der zu analysierenden Komponenten 13 CO2 und 12 CO2 bis zu 20 delta‰ über den Dynamikbereich ber 12 C02 - Konzentration. Bei einer gewünschten Toleranz von < 0,3 delta‰ ist diese Abweichung zu groß um eine sichere Atemgasdiagnose durchführen zu können.

Weiterhin sind Korrekturverfahren bekannt, bei welchen bei einem Ausgangsgasgemisch Umgebungsluft zudosiert wird. Die Verdünnungskurve wird ermittelt und abgespeichert. Der beim Atemtest erzeugte delta- oder R-Wert wird über den Dynamikbereich der 12C02 Konzentration mit der Verdünnungskurve korrigiert.

Bei dieser Methode wird die exakte Korrektur jedoch nicht erreicht, da das fremdzugemischte Gas mit Umgebungsbedingungen z.B. einen zur Expirationsluft des Patienten unterschiedliche Wasserdampfgehalt hat damit einen Volumenfehler erzeugt. Hinzu kommt. dass die nicht sicher reproduzierbare C02-Konzentration der Atmosphäre die Korrekturkurve verfälscht.

Eine Korrektur nach diesem Verfahren ist ausreichend, wenn die Gasprobe aus einem vorgegebenen Reservoir entnommen wird.

Der Erfindung liegt daher die Aufgabe zugrunde, ein Verfahren sowie eine Einrichtung der gattungsgemäßen Art dahingehend zu verbessern, daß trotz Trägergasabhängigkeit mit einer noch höheren Genauigkeit und Reproduzierbarkeit gemessen werden kann.

Die gestellte Aufgabe wird bei einem Verfahren der gattungsgemäßen Art erfindungsgemäß durch die kennzeichnenden Merkmale des Patentanspruches 1 gelöst.

Weitere vorteilhafte Ausgestaltungen des erfindungsgemäßen Verfahrens sind in den abhängigen Ansprüchen dargestellt.

Kern der verfahrensgemäßen Erfindung ist, daß in einem 1. Verfahrensschritt Atemgasteilproben mit unterschiedlicher Konzentration der zu messenden Gaskomponente und entsprechend der Trägergaskomponente durch unterschiedliche Atemtiefe erzeugt werden, und daß in einem 2. Verfahrensschritt eine Korrekturkurve aus den unterschiedlichen Werten der verschiedenen Atemgasteilproben ermittelt wird, und im nachfolgenden Messlauf die so erhaltene Korrekturkurve zur Eliminierung der Trägergasabhängigkeit herangezogen wird.

Damit ist es nunmehr möglich, aus dem Messgas, hier aus der Expirationsluft, d.h. aus seiner variierenden Zusammensetzung heraus eine Kalibrierung vorzunehmen, ohne separates Kalibriergas verwenden zu müssen. Da bei der Expirationluft von Patienten die immer wieder zueinander unterschiedlichen Atemtiefen die Trägergaszusammensetzung variieren läßt, bewirkt die zuvorige Aufnahme einer Korrekturkurve bei unterschiedlichen Atemtiefen, dass man für die gesamte Bandbreite der Trägergasvarianz eine aktuelle Korrekturkurve für die gesamte nachfolgende Messung vorliegen hat.

Besonders beachtlich sind die mit dieser nur einfach scheinenden Maßnahme erhaltenen vorteilhaften Wirkungen. So ist neben der sauerstoffbedingten Trägergasabhängigkeit auch ein Effekt bezüglich des Wasserdampfes, der in der Expirationsluft immer enthalten ist, bekannt. Dies betrifft in erster Linie nicht nur die sogenannte Wasserdampfquerempfindlichkeit, sondern auch Kondensationen von Wasserdampf entlang des Weges, den das Messgas innerhalb der Apparatur läuft. Aufgrund von Temperaturunterschieden kann der Wasserdampf der Expirationsluft in der Apparatur kondensieren und somit die Partialdruckverhältnisse ad hoc verändern. Hierdurch verändern sich ad hoc auch die daraus resultierenden Ergebnisse hinsichtlich der Trägergasabhängigkeit, weil die sich daraufhin einstellenden Partialdrücke wieder anders sind. Diesem Effekt der Unbestimmbarkeit begegnet die erfindungsgemäße Vorgehensweise auf besonders wirkunksvolle Weise. Bei der Aufnahme der Kalibrierkurve kurz vor der Messung werden die Trägergasabhängigkeiten bei unterschiedlichen Konzentrationen sowie auch Wasserdampfeffekte samt apparativ bedingter Kondensationseffekte "in einem Zuge" berücksichtigt und in der "unmittelbar" nachfolgenden Messung eliminiert werden. Auf diese erfindungsgemäße Weise ist es erstmalig möglich tatsächlich eine Online-Messung auch der stoffwechselbezogenen Vitalfunktionen vornehmen zu können. Übrige bisher bekannte Verfahren nehmen entweder die besagten Fehler in Kauf, wodurch das Meßergebnis ohne faktische Aussagekraft ist, oder setzen eine separate Kalibrierung voraus, so daß eine tatsächliche Online-Messung nicht möglich ist.

In vorteilhafter Ausgestaltung des erfindungsgemäßen Verfahrens ist angegeben, daß die Atemgasprobe aus einem Reservoir entnommen wird. Hierdurch wird die Messung unabhängig von der Anwesenheit eines Patienten, der lediglich zur diagnostischen Zwecken eine Atemgasprobe vorort beläßt.

Eine weiterhin vorteilhafte Ausgestaltung besteht darin, daß die Atemgasproben vom Probanden direkt auf das Spektrometer gegeben werden. Hierbei können durch unterschiedliche Atemtiefen Meßwerte mit impliziter Trägergasabhängigkeit bei unterschiedlichen Sauerstoffanteilen in der Expirationsluft aufgenommen werden. Es bedarf also keiner Kalibrierung mittels eines Kalibriergases, sondern die Kalibrierung erfolgt über unterschiedliche Sauerstoffpartialdrücke in der Expirationsluft. Dabei bewirkt ein tiefer Atemzug, daß ein deutlich niedriger Partialdruckanteil von Sauerstoff in der Expirationsluft enthalten ist, als bei einem flachen Atemzug. Da die direkte Expirationsluft auch Wasserdampf enthält, kann eine entsprechende Berücksichtigung sofort vorort und für die nachfolgende Messung maßgebend, erfolgen.

Auf diese Weise erfolgt eine Kalibrierung direkt durch den Probanden, und die zuvorige Kalibrierphase, bspw durch mehrere Atemzüge unterschiedlicher Atemtiefe, geht für den Probanden unbemerkt und fließend in die Meßphase über.
Im übrigen ist die direkte Kalibrierung mittels der originären Expirationsluft unterschiedlicher Atemtiefe eine Kalibrierung in Berücksichtigung bereits der originären Meßgasmatrix, in welcher nunmehr auch die Varianz der Partialdruckverhältnisse verschiedener Trägergaskomponenten berücksichtigt ist.

Dabei ist in vorteilhafter Ausgestaltung angegeben, daß sowohl die Kalibrierung als auch die nachfolgende Messung bei gleicher Umgebungstemperatur, vorzugsweise bei Raumtemperatur vorgenommen wird. Dies hat unter anderem den Vorteil, daß etwaige Kondensationseffekte sowie Volumen- und damit ggfs temperaturabhängige Partialdruckeffekte bereits in der zuvor aufgenommenen Korrekturkurve berücksichtigt sind.

In weiterer vorteilhafter Ausgestaltung ist angegeben, daß nach erfolgter Aufnahme der Korrekturkurve die Expirationsluft eines Probanden online und sukzessive mehrerer aufeinanderfolgender Atemzüge in ein Mischvolumen eingebracht wird, und daß nach einer vorgebbaren Wartezeit aus dem Mischvolumen Probengas in das Spektrometer eingebracht wird. Hierdurch ergibt sich eine gewisse Integration über nur kurzzeitige Varianzeffekte in dem Probengas. Von der Größe des Mischvolumens hängen jedoch die Zeitkonstanten ab, in welchen bei einer online-Messung am Patienten die Änderungen der aus der Gasanalyse abgeleiteten Vitalfunktionen bestimmbar ist.

Ein Mischvolumen, ggfs auch größeren Volumens empfiehlt sich dadurch insbesondere dann, wenn aus der Expirationsluft ein quasi stationärer Zustand, wie das Vorliegen bspw einer helicobacter pylori Infektion bestimmt werden soll.

Im Hinblick auf die Einrichtung insbesondere zur Durchführung des erfindungsgemäßen Verfahrens, wird die gestellte Aufgabe erfindungsgemäß durch die Vorsehung eines Mischvolumens gelöst, welches als gasdynamische Vorstufe dem Spektrometer, d.h. der Meßküvette vorgeschaltet ist. Da dieses Mischvolumen natürlich nicht evakuiert ist, ist dort ein Mischgas bekannter zusammensetzung enthalten, in welches das Meßgas ohne wesentliche Absolutdruckerhöhung hinzumischbar ist. Über diesen Mischprozeß ist die Totzeit beeinflußbar, und nach einer entsprechenden Totzeit wird die Messung begonnen.

Vorteilhaft, wenn auch nicht zwingend ist, daß das Mischvolumen gegen andere Mischvolumen anderen Volumens austauschbar ist.

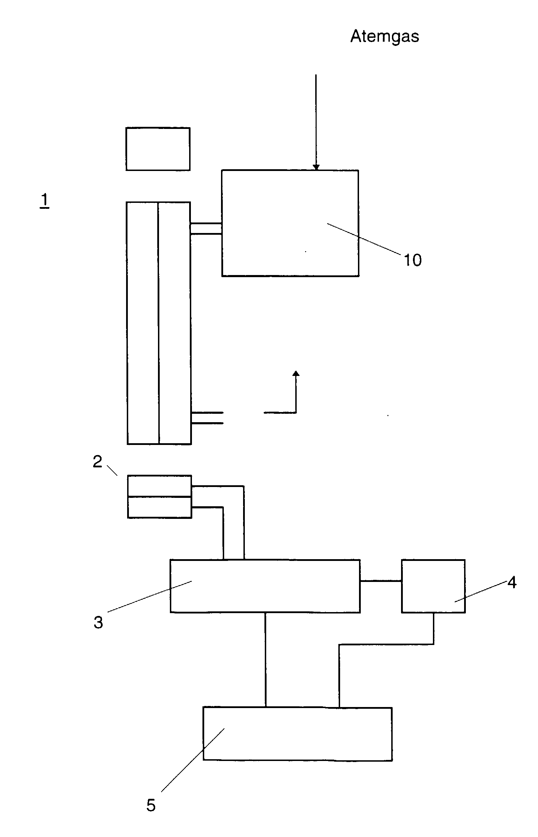

Die Abbildung zeigt den funktionalen Zusammenhang der verfahrensgemäßen Maßnahmen.

Das Meßgas wird in dem hier dargestellten Beispiel über ein der Meßküvette 1 eines nichtdispersiven Infrarotspektrometers vorgeschaltetes Mischvolumen 10 zugeführt. Die Zuführung kann dabei jedoch auch ohne Mischvolumen erfolgen. Gemäß dem erfindungsgemäßen Verfahren werden nun Atemgasproben unterschiedlicher Atemtiefe am Beginn der Messung zugeführt. Hierdurch entstehen Atemgase mit unterschiedlichem Sauerstoffpartialdruck und somit unterschiedliche Trägergasabhängigkeiten. Diese werden von dem Detektor erfasst und als Meßsignale einer Meßsignalauswertung 3 zugeführt. In einer Korrektureinheit 4 werden die aufgrund der unterschiedlichen Atemtiefen und dadurch bewirkten unterschiedlichen Sauerstoffpatialdrücke der Expirationsluft eine Korrekturkurve im Hinblick auf die dadurch bewirkte unterschiedliche Trägergasabhängigkeit erstellt. Der nachfolgenden Anzeigeeinheit 5 werden somit der aufbereitete Meßwert und der entsprechende Korrekturwert aus der Korrekturkurve zugeführt. In der Anzeigeeinheit werden die Werte entsprechend korrigiert und der korrigierte Wert als Meßwert angezeigt bzw ausgewertet.

## Patentansprüche

1. Verfahren zur Kalibrierung einer Meßeinrichtung, insbesondere eines nichtdispersiven Infrarotspektrometers, zur Atemgasanalyse, mit einem Strahlengang, bspw mit einem 12CO₂ und 13CO₂ Kanal, in welchem die besagten Gaskomponenten selektiv gemessen werden können,
dadurch gekennzeichnet,
daß in einem 1. Verfahrensschritt Atemgasteilproben mit unterschiedlicher Konzentration der zu messenden Gaskomponente und entsprechend der Trägergaskomponente durch unterschiedliche Atemtiefe erzeugt werden, und daß in einem 2. Verfahrensschritt eine Korrekturkurve aus den unterschiedlichen Werten der verschiedenen Atemgasteilproben ermittelt wird, und im nachfolgenden Messlauf die so erhaltene Korrekturkurve zur Eliminierung der Trägergasabhängigkeit herangezogen wird.

2. Verfahren nach Anspruch 1,
dadurch gekennzeichnet,
daß die Atemgasprobe aus Reservoir entnommen wird.

3. Verfahren nach Anspruch 1,
dadurch gekennzeichnet,
daß die Atemgasproben vom Probanden direkt auf das Spektrometer gegeben werden.

4. Verfahren nach einem der vorhergehenden Ansprüche,
dadurch gekennzeichnet,
daß sowohl die Kalibrierung als auch die nachfolgende Messung bei gleicher Umgebungstemperatur, vorzugsweise bei Raumtemperatur durchgeführt wird.

5. Verfahren nach einem oder mehreren der vorhergehenden Ansprüche,
dadurch gekennzeichnet,
daß die Expirationsluft eines Probanden online und sukzessive mehrere aufeinanderfolgende Atemzüge in ein Mischvolumen eingebracht wird, und dass nach einer vorgebbaren Wartezeit aus dem Mischvolumen Probengas in das Spektrometer eingebracht wird.

6. Einrichtung zur Kalibrierung einer Meßeinrichtung, insbesondere eines nichtdispersiven Infrarotspektrometers, zur Atemgasanalyse, mit einem Strahlengang, bspw mit einem 12CO₂ und 13CO₂ Kanal, in welchem die besagten Gaskomponenten selektiv messbar sind,
dadurch gekennzeichnet,
daß dem Spektrometer (1) eine gasdynamische Vorstufe mit einem vorgegebenen oder einem vorgebbaren Mischvolumen (10) vorgeschaltet ist, welches vor Beginn der Messung mit einem Mischgas bekannter Zusammensetzung gefüllt ist, in welches sodann ohne wesentliche Druckerhöhung das Messgas hinzumischbar und nach einstellbarer Totzeit die Messung online begonnen wird.

7. Einrichtung nach Anspruch 7,
dadurch gekennzeichnet,
daß das Mischvolumen (10) gegen andere Mischvolumen unterschiedlichen Volumens austauschbar ist.
